Europäisches Patentamt

European Patent Office

Office européen des brevets

(19)

(11)  EP 0 598 329 B1

(12)  **EUROPÄISCHE PATENTSCHRIFT**

(45) Veröffentlichungstag und Bekanntmachung des Hinweises auf die Patenterteilung:
**06.02.2002   Patentblatt 2002/06**

(51) Int Cl.$^7$: **G01N 33/72**, C07K 16/18

(21) Anmeldenummer: **93118251.3**

(22) Anmeldetag: **11.11.1993**

(54) **Simultane Bestimmung von HbA1c und Hämoglobinvarianten mit einer HbA1c-analogen Glykierung**

Simultaneous determination of HbA1c and haemoglobin variants with a HbA1c analog glycation

Détermination simultanée de HbA1c et des variantes de l'hémoglobine avec une glycération analogue à HbA1c

(84) Benannte Vertragsstaaten:
**AT BE CH DE DK ES FR GB GR IE IT LI LU NL PT SE**

(30) Priorität: **17.11.1992  DE 4238705**
**31.03.1993  DE 4310500**

(43) Veröffentlichungstag der Anmeldung:
**25.05.1994   Patentblatt 1994/21**

(73) Patentinhaber: **Roche Diagnostics GmbH**
**68305 Mannheim (DE)**

(72) Erfinder:
• **Karl, Johann, Dr.**
  **D-82380 Peissenberg (DE)**
• **Finke, Andreas, Dr.**
  **D-82407 Wielenbach (DE)**
• **Engel, Wolf-Dieter, Dr.**
  **D-82340 Feldafing (DE)**

(56) Entgegenhaltungen:
**EP-A- 0 201 187          EP-A- 0 329 994**
**DE-A- 3 439 610          US-A- 4 478 744**

**Beschreibung**

Gegenstand der Erfindung ist ein Verfahren zur immunologischen Bestimmung des Gehaltes an glykiertem Hämoglobin in einer Blutprobe, bei welchem man Antikörper verwendet, die $HbA1_c$, $HbS1_c$ und $HbC1_c$ erkennen, sowie ein Verfahren zur Herstellung solcher Antikörper.

Hämoglobin, das den Transport von Sauerstoff und $CO_2$ bewirkt und in den Erythrozyten lokalisiert ist, besteht aus vier Proteinketten, von denen jeweils zwei die gleiche Struktur haben. Überwiegend besteht es aus jeweils zwei nicht glykosylierten $\alpha$- und $\beta$-Ketten. Das Hämoglobin liegt im Blut normalerweise zu mehr als 90 % in dieser als $HbA_0$ bezeichneten Form vor.

Sowohl von der $\alpha$- als auch von der $\beta$-Kette des Hämoglobins sind Varianten mit einer veränderten Aminosäuresequenz bekannt, die die Transportfunktion des Hämoglobinmoleküls beeinträchtigen und zu sogenannten Hämoglobinopathien führen. Bei der bekanntesten dieser Hämoglobinopathien, der Sichelzellanämie, ist in Position 6 der $\beta$-Kette die hydrophile Glutaminsäure durch das hydrophobe Valin ersetzt. Hierdurch kommt es zu einer hydrophoben "Zyklisierung" zwischen diesem Valin und dem Valin in Position 1 der $\beta$-Kette. Durch diese Struktur wird eine Aggregation der eine solche veränderte $\beta$-Kette enthaltenden HbS-Moleküle bewirkt, welche die Transportfunktion dieses veränderten Hämoglobins beeinflußt. Auch das HbC, ein anderes pathologisches Hämoglobin, unterscheidet sich vom HbA durch einen Aminosäureaustausch in Position 6 der $\beta$-Kette. In diesem Fall ist die Glutaminsäure durch Lysin ersetzt. Daneben sind noch eine Vielzahl weiterer Hämoglobinvarianten mit einem definierten Aminosäureaustausch bekannt (z.B. HbA2, HbE).

Von diesen Hämoglobinvarianten werden in vivo durch nicht enzymatische Reaktion mit Glucose glykierte Hämoglobinderivate gebildet. Diese nicht enzymatische Glykierung ist eine langsam, kontinuierlich und irreversibel ablaufende Reaktion, die im wesentlichen von der Blutglucosekonzentration abhängig ist. Die Glykierung verläuft über die Bildung einer Schiff'schen Base zwischen der Aldehydgruppe der Glucose und der freien Aminosäure des Hämoglobins. Das hierbei gebildete Aldimin lagert sich durch eine Amadori-Umlagerung zum N-(1-Desoxy-D-fructos-1-yl)-Rest um. In dieser umgelagerten Form ist das glykierte Hämoglobin stabil.

Das üblicherweise entstehende glykierte Hämoglobin wird mit $HbA1_c$ bezeichnet. Die oben genannten glykierten Hämoglobinvarianten werden mit $HbS1_c$ oder $HbC1_c$ bezeichnet. Diese entstehen durch Glykierung der freien Aminogruppe des Valin- bzw. Lysinrestes, der sich am N-terminalen Ende der $\beta$-Kette des Hämoglobins befindet. Dabei werden die verschiedenen Hämoglobinvarianten in gleichem Ausmaß glykiert wie das normale $HbA_0$ (J. Sosenko et al., Diabetes Care 3 (1980), 590 - 593).

Die Konzentration der glykierten Hämoglobine im Blut ist abhängig von der Blutglucosekonzentration. Der Anteil der glykierten Hämoglobine am Gesamthämoglobin liegt bei Erwachsenen normalerweise im Bereich von 3 - 6 %. Bei erhöhtem Blutzuckerspiegel nimmt dieser Anteil bis auf 15 % zu. Die Bestimmung des Anteils an N-terminal glykiertem Hämoglobin ist daher ein zuverlässiger Parameter zur Kontrolle des Blutglucosespiegels. Da die Erythrozyten eine durchschnittliche Halbwertszeit von 120 Tagen aufweisen, bietet die Bestimmung von glykiertem Hämoglobin im Blut einen Parameter für den Blutglucosespiegel, der unabhängig von einer kurzzeitigen Erhöhung, z.B. nach einer kohlehydratreichen Mahlzeit, ist.

Die Bestimmung der glykierten Hämoglobine im Blut ist daher von großer Bedeutung für die Diagnose und Überwachung eines Diabetes mellitus. Es sind daher eine Reihe von chromatographischen und elektrophoretischen Methoden zum Nachweis von $HbA1_c$ entwickelt worden. Die glykierten Hämoglobinvarianten lassen sich mit diesen Methoden dagegen nicht simultan mit $HbA1_c$ bestimmen (J. Sosenko et al., Diabetes Care 3 (1980), 590 -593, D. Goldstein et al., CRC Critical Reviews in Clinical Laboratory Sciences 21 (1984), 187 - 225 und Allen et al., Annual Clinical Biochemistry 29 (1992), 426 - 429). Mit Hilfe der affinitätschromatographischen Methoden werden zwar auch die glykierten Hämoglobinvarianten wie $HbS1_c$ oder $HbC1_c$ neben $HbA1_c$ simultan bestimmt, doch ist diese Methode nicht spezifisch für die Glykierung am N-Terminus der $\beta$-Kette, da alle Glykierungen des Hämoglobinmoleküls (z.B. an Lysin-Resten oder an der $\alpha$-Kette) gleich erfaßt werden (D. Goldstein et al., CRC Critical Reviews in Clinical Laboratory Sciences 21 (1984), 187 - 225). Dadurch ergeben die chromatographischen Verfahren bei einem Patienten mit einer der oben genannten Hämoglobinopathien zu geringe Werte für den Anteil an N-terminal glykiertem Hämoglobin, während die affinitätschromatographischen Methoden allgemein höher messen. Daher sind diese bekannten Verfahren zur Diagnose und Überwachung eines Diabetes mellitus bei Blutproben von Patienten mit Hämoglobinopathien nicht anwendbar.

In EP-A 0 329 994 und DE-A 34 39 610 werden Immunogene sowie Verfahren zur Herstellung von Antikörpern gegen glykolysiertes Hämoglobin beschrieben.

Es hat sich jetzt überraschenderweise gezeigt, daß mit Antikörpern, die erhältlich sind durch Immunisierung mit einem Immunogen, welches als Haptenteil das glykierte Oligopeptid Fructose-Val-His-Leu-Thr-Pro oder einen Teil davon enthält, Antikörper erhalten werden, die $HbA1_c$, $HbS1_c$ und $HbC1_c$ erkennen und für die Verwendung in immunologischen Bestimmungsverfahren für $HbA1_c$, $HbS1_c$ und $HbC1_c$ geeignet sind.

Gegenstand der Erfindung ist demnach die Verwendung von Antikörpern, die $HbA1_c$, $HbS1_c$ und $HbC1_c$ erkennen und erhältlich sind durch Immunisierung mit mindestens einem Immunogen, welches als Hapteil das glykierte Oligopeptid Fructose-Val-His, Fructose-Val-His-Leu, Fructose-Val-His-Leu-Thr und/oder Fructose-Val-His-Leu-Thr-Pro enthält zur immunologischen, simultanen Bestimmung von $HbA1_c$, $HbS1_c$ und $HbC1_c$.

Daß derartig hergestellte Antikörper $HbA1_c$, $HbS1_c$ und $HbC1_c$ erkennen, ist insbesondere auch deshalb überraschend, weil der Aminosäureaustausch in Position 6 der β-Kette von HbS und HbC auch zu einer Änderung der Tertiärstruktur im Bereich des vom Antikörper erkannten Epitops führt und demzufolge zu erwarten gewesen wäre, daß Antikörper, die mit dem oben genannten Immunogen erhalten werden, $HbA1_c$, $HbS1_c$ und $HbC1_c$ differenzieren.

Mit diesen Antikörpern kann die simultane immunologische Bestimmung von N-terminal glykiertem Hämoglobin $HbA1_c$, $HbS1_c$ und $HbC1_c$ über alle gängigen Immunoassays wie z.B. ELISA, Fluoreszenzimmunoassay, Radioimmunoassay, Fluoreszenz-Polarisations-Immunoassay (FPIA), Cloned Enzyme Donor Immunoassay (CEDIA®) oder Enzyme-Multiplied-Immunoassay-Technique (EMIT) erfolgen. Der Test kann dabei sowohl als homogener Test, z.B. als ein kompetitiver turbidimetrischer Immunoassay, als auch als heterogener Test durchgeführt werden.

Vorzugsweise erfolgt der Test nach dem Prinzip des Agglutinationstests, wie z.B. TINIA oder dem Latex Particle-Enhanced Immunoassay (LPIA). Hierbei handelt es sich um einen kompetitiven Test, bei welchem das Antigen aus der Probe mit einem Polyhapten, in dem mehrere Haptene an ein hochmolekulares Trägerprotein gebunden vorliegen, um die Bindung an einen spezifischen Antikörper konkurriert. Vorzugsweise wird als Hapten, das an einen hochmolekularen Träger gebunden vorliegt, das zur Immunisierung verwendete Hapten (vorzugsweise Fructose-Val-His-Leu-Thr-Pro) verwendet. In Abwesenheit von Antigen aus der Probe wird dieses Immunogen durch den im Test eingesetzten Antikörper zu großen Aggregaten vernetzt, welche eine bestimmte Trübung dieser Testlösung verursachen. Entsprechend der Menge an Antigen in der Probe wird hochmolekulares Polyhapten aus diesen Aggregaten durch das Antigen verdrängt. Damit nimmt die Trübung in einer zur Antigenmenge proportionalen Weise ab. Durch Vergleich mit der Trübungsabnahme, die bei Zugabe bekannter Mengen an glykiertem Hämoglobin beobachtet wird, kann die Menge an glykiertem Hämoglobin ($HbA1_c$, $HbS1_c$ und $HbC1_c$) in der Probelösung bestimmt werden. Als Standard können dabei $HbA1_c$, $HbS1_c$ oder $HbC1_c$ allein oder als Gemische verwendet werden.

Weiterhin bevorzugt ist die Durchführung des erfindungsgemäßen Verfahrens nach dem Prinzip des CEDIA®, EMIT, FPIA oder ELISA.

Beim CEDIA®-Prinzip bewirkt das Antigen aus der zu analysierenden Probe die Assoziation von allein jeweils inaktivem Enzymakzeptor und Enzymdonor zu einem aktiven Enzym, dessen Aktivität somit proportional zur Menge an Antigen in der zu analysierenden Probe ist (Henderson et al., Clinical Chemistry 32 (1986), 1637 - 1641). Zum Nachweis werden hierbei bestimmte Enzyme wie z.B. die β-Galactosidase verwendet, die in zwei jeweils enzymatisch inaktiven Bestandteilen, nämlich einem großen Polypeptid (Enzymakzeptor) und einem kleinen Polypeptid (Enzymdonor) vorliegen, wobei diese Bestandteile spontan zu einem enzymatisch aktiven Protein assoziieren. An den Enzymdonor wird das als Analyt nachzuweisende Hapten derart gebunden, daß die Assoziation des Enzymdonors mit dem Enzymakzeptor zum aktiven Enzym durch diese Bindung nicht verhindert wird. Diese Assoziation wird aber dann gehemmt, wenn an den Antigen-Enzymdonor-Komplex ein Antikörper gegen das Antigen bindet. In einer Reagenzlösung, in der Enzymakzeptor, Antigen-Enzymdonor-Komplex und der entsprechende Antikörper vorliegen, kann daher kein aktives Enzym gebildet werden und es wird keine enzymatische Aktivität gemessen. Nach Zugabe der Probelösung verdrängt nun das Antigen aus dieser Probelösung den Antikörper aus der Bindung an den Antigen-Enzymdonor-Komplex und ermöglicht so die Ausbildung des aktiven Enzyms.

Bei der Enzyme Multiplied Immunoassay Technique (EMIT) wird das nachzuweisende Hapten so kovalent mit dem Markerenzym gekoppelt, daß die Enzymaktivität erhalten bleibt. Nach Bindung eines Antikörpers an den Haptenanteil wird die Substratbindung an das Enzym sterisch jedoch behindert, so daß keine enzymatische Umsetzung des Substrats erfolgen kann. Wie beim CEDIA-Prinzip verdrängt dann auch hier das Antigen aus der zu bestimmenden Probelösung den Antikörper vom enzymgebundenen Hapten und ermöglicht so eine enzymatische Aktivität, die proportional zur Konzentration des zu analysierenden Antigens in der Probelösung ist (Gunzer et al., Kontakte III, 1980, 3 - 11 und K. Rubenstein, Biochemical and Biophysical Research Communications 47 (1972), 846 - 851).

Beim Fluoreszenz-Polarisations-Immunoassay (FPIA) wird das zu bestimmende Hapten mit einer fluoreszierenden Substanz markiert. Diese Moleküle absorbieren Lichtenergie und geben sie in einem Zeitraum von etwa $10^{-8}$ sec als Licht längerer Wellenlänge wieder ab. Wird der Fluorophor durch polarisiertes Licht angeregt, so hängt der Polarisationsgrad des emittierten Lichts von der Rotationsgeschwindigkeit des Tracers (Analyt-Fluorophor-Konjugat) ab. Die Bindung des Tracers an einen Antikörper behindert die Rotation des Fluorophors. Der freie Tracer rotiert schneller und depolarisiert das anregende Licht mehr als der größere, trägere Antikörper-Tracer-Komplex. Je mehr der Analyt in der Probe vorhanden ist, desto weniger Antikörper-Tracer-Komplexe entstehen und desto weniger Fluoreszenzpolarisation ist meßbar (W. Dandliker et al., Journal of Exp. Med. 122 (1965), 1029).

Bei Immunoassays nach dem ELISA-Prinzip wird die Bindung eines enzymmarkierten Antikörpers an ein vor oder während der Nachweisreaktion immobilisiertes Antigen aus der zu bestimmenden Probelösung über die Messung der Enzymmarkierung in der festen Phase bestimmt.

Ein weiterer Gegenstand der Erfindung sind monoklonale und polyklonale Antikörper, die $HbA1_c$, $HbS1_c$ und HbC1$_c$ erkennen und erhältlich sind durch Immunisierung mit einem Immunogen, das als Hapten das glykierte Oligopeptid Fructose-Val-His, Fructose-Val-His-Leu oder Fructose-Val-His-Leu-Thr-Pro enthält, und Isolierung des Antikörpers aus dem Serum der immunisierten Tiere nach bekannten Verfahren.

Ein weiterer Gegenstand sind monoklonale und polyklonale Antikörper, erhältlich durch Immunisierung eines Säugers mit einem Gemisch aus einem Immunogen, das als Hapten Fructose-Val-His-Leu-Thr enthält und mindestens einem weiteren Immunogen, welches als Haptenteil Fructose-Val-His, Fructose-Val-His-Leu und/oder Fructose-Val-His-Leu-Thr-Pro enthält, und Isolierung des Antikörpers aus dem Serum der immunisierten Tiere.

Ein bevorzugter Gegenstand der Erfindung sind monoklonale Antikörper, die HbA1$_c$, HbS1$_c$ und HbC1$_c$ erkennen und erhältlich sind durch Immunisierung mit dem genannten Immunogen, Immortalisierung der Milzzellen der immunisierten Tiere, Klonierung derjenigen immortalisierten Zellen, die den gewünschten Antikörper produzieren, und Isolierung des Antikörpers aus den Antikörper produzierenden Zellen nach bekannten Verfahren.

Das Immunogen kann analog den in EP-A 0 329 994 (die Gegenstand der Offenbarung der vorliegenden Patentanmeldung ist) beschriebenen Verfahren hergestellt werden. Das Hapten wird dabei an ein Trägerprotein wie z.B. KLH (keyhole limpet hemocyanin), β-Galactosidase oder Edestin gebunden. Vorzugsweise wird KLH als Trägerprotein verwendet. Die Kopplung zwischen Hapten und Trägerprotein erfolgt zweckmäßig über Kopplungsgruppen wie z.B. Lysin, Cystein und die Maleinimidohexylgruppe.

Besonders bevorzugt wird ein Immunogen verwendet, welches eine hohe Beladungsdichte aufweist (Anzahl der gebundenen Haptengruppen entsprechend 5 - 25 % des Gewichts des Trägerproteins). Dabei wird ein Antiserum hoher Serumstärke erhalten. Unter einer hohen Serumstärke ist zu verstehen, daß das erhaltene polyklonale Antiserum im Reagenz zur Bestimmung der glykierten Hämoglobine in hohen Verdünnungen (1:8, vorzugsweise 1:10 und mehr) eingesetzt werden kann.

Mit diesem Immunogen werden dann die üblicherweise zur Gewinnung von Antikörpern verwendeten Tiere nach dem Fachmann bekannten Verfahren immunisiert. Vorzugsweise werden Kaninchen oder Schafe bzw. zur Herstellung von monoklonalen Antikörpern Mäuse verwendet. Die polyklonalen Antikörper können entweder direkt oder vorzugsweise nach chromatographischer Reinigung an DEAE oder immunsorptiver Reinigung verwendet werden. Monoklonale Antikörper werden in üblicher Weise durch Immortalisierung der Milzzellen der immunisierten Tiere, Klonierung derjenigen immortalisierten Zellen, die den gewünschten Antikörper produzieren, und Isolierung des Antikörpers nach bekannten Verfahren erhalten.

Ebenso ist es bevorzugt, zur Immunisierung ein Gemisch von mindestens zwei Immunogenen zu verwenden, die als Haptenteil Fructose-Val-His, Fructose-Val-His-Leu, Fructose-Val-His-Leu-Thr und Fructose-Val-His-Leu-Thr-Pro enthalten.

Diejenigen immortalisierten Zellen, die den gewünschten Antikörper produzieren, werden in üblicher Weise über einen ELISA-Test zum Nachweis der Bindung an BbA1$_c$, HbS1$_c$ und gegebenenfalls HbC1$_c$ identifiziert.

Ein weiterer Gegenstand der Erfindung ist ein Verfahren zur Herstellung von Antikörpern, die sowohl HbA1$_c$ als auch HbS1$_c$ und HbC1$_c$ erkennen, durch Immunisierung mit einem Immunogen, das als Hapten das glykierte Oligopeptid Fructose-Val-His, Fructose-Val-His-Leu oder Fructose-Val-His-Leu-Thr-Pro enthält, oder einem Gemisch von mindestens zwei solchen Immunogenen, und Isolierung des Antikörpers aus dem Serum der immunisierten Tiere nach bekannten Verfahren.

Ein bevorzugter Gegenstand der Erfindung ist ein Verfahren zur Herstellung von monoklonalen Antikörpern, die HbA1$_c$, HbS1$_c$ und HbC1$_c$ erkennen, durch Immunisierung mit einem Immunogen, das als Hapten das glykierte Oligopeptid Fructose-Val-His, Fructose-Val-His-Leu oder Fructose-Val-His-Leu-Thr-Pro enthält, oder einem Gemisch von mindestens zwei solchen Immunogenen, Immortalisierung der Milzzellen der immunisierten Tiere, Klonierung derjenigen immortalisierten Zellen, die den gewünschten Antikörper produzieren, und Isolierung des Antikörpers aus den klonierten Zellen nach bekannten Verfahren.

Ein besonders bevorzugter Gegenstand der Erfindung ist ein solches erfindungsgemäßes Verfahren zur Herstellung von polyklonalen oder monoklonalen Antikörpern, die HbA1$_c$, HbS1$_c$ und HbC1$_c$ erkennen, bei welchem ein Immunogen verwendet wird, bei dem der Haptenteil an KLH als Trägerprotein gebunden ist.

Ein weiterer Gegenstand der Erfindung ist die Verwendung eines erfindungsgemäßen Antikörpers zur immunologischen Bestimmung des Gehaltes an glykiertem Hämoglobin in einem Verfahren zur simultanen immunologischen Bestimmung des Gehaltes an HbA1$_c$ sowie glykierten Hämoglobinvarianten wie z.B. HbS1$_c$ und HbC1$_c$ in einer Blutprobe.

Die erfindungsgemäßen Antikörper erkennen zusätzlich noch weitere Glykierungsvarianten von Hb wie z.B. HbA2$_{1c}$ und HbE1$_c$.

Ein weiterer Gegenstand der Erfindung ist ein Reagenz zur immunologischen Bestimmung des Gehaltes an N-terminal glykiertem Hämoglobin, welcher mindestens einen erfindungsgemäßen Antikörper enthält, sowie ein Verfahren zur immunologischen, simultanen Bestimmung von HbAl$_c$, HbSl$_c$ und HbCl$_c$ unter Verwendung der erfindungsgemäßen Antikörper.

Die Erfindung wird durch die folgenden Beispiele näher erläutert.

**Beispiel 1:**

**Herstellung von Immunogenen zur Gewinnung von Antikörpern gegen glykierte Hämoglobine vom HbA1$_c$-Typ**

**1.1 Verwendung von β-Galactosidase als Trägerprotein**

Das Peptid Fructose-Val-His-Leu-Thr-Lys-OH wird gemäß den Angaben in EP-A 0 329 994 über eine Festphasensynthese an einem semiautomatischen Peptidsynthesizer der Firm Labortec (Bubendorf, Schweiz) synthetisiert und an β-Galactosidase gebunden. Dazu werden 10 mg Fructose-Val-His-Leu-Thr-Lys-OH in 1 ml 0,1 mol/l Kaliumphosphatpuffer pH 7 aufgenommen und 6,2 mg Maleinimidohexansäure-N-hydroxysuccinimidester in 2 ml Ethanol zugegeben. Die Reaktionslösung wird 14 h bei Raumtemperatur gerührt und anschließend an Polycosil C18 aufgereinigt und die gemäß HPLC reinen Fraktionen lyophilisiert.

Zur Herstellung des Immunogens werden 48 mg β-Galactosidase (β-Gal) (EIA-Qualität, Boehringer Mannheim GmbH, Kat.-Nr. 570 079) unter Argonatmosphäre in 2 ml mit Argon-begastem 0,1 mol/l Kaliumphosphatpuffer pH 7,0 gelöst und unter Sauerstoffausschluß 5 mg des lyophilisierten Peptids Fructose-Val-His-Leu-Thr-Lys(MH)-OH zugegeben und 1 h bei Raumtemperatur gerührt. Anschließend wird die gesamte Reaktionslösung auf eine AcA202-Säule (2 x 24 cm, Pharmacia, Schweden), welche mit Argon-gesättigter 0,09%iger Natriumchloridlösung equilibriert wurde, aufgetragen. Anschließend wird mit dem gleichen Equilibrierungspuffer eluiert und die Proteinfraktionen, die das gewünschte Immunogen enthalten, gesammelt. Die Beladung der β-Galactosidase mit den Haptengruppen kann durch Umsetzung einer Probe mit Ellmanns Reagenz bestimmt werden.

In analoger Weise werden die Immunogene Fructose-Val-His-Lys-(MH)-βGal, Fructose-Val-His-Leu-Lys-(MH)-βGal und Fructose-Val-His-Leu-Thr-Pro-Lys-(MH)-βGal hergestellt.

**1.2 Verwendung von KLH als Trägerprotein**

Das Hapten Fructose-Val-His-Leu-Thr-Cys-OH wird gemäß den Angaben in EP-A 0 329 994 über eine Festphasensynthese an einem semiautomatischen Peptidsynthesizer der Firm Labortec (Bubendorf, Schweiz) synthetisiert.

Das Trägerprotein Keyhole Limpet Hämocyanin (KLH) wird mit Maleinimidohexanoyl-N-hydroxysuccinimid (MHS) umgesetzt. Dazu werden 2 g KLH in 70 ml 0,1 mol/l Natriumhydrogencarbonat pH 8,35 gelöst. Der unlösliche Proteinanteil wird durch Zentrifugation abgetrennt. Anschließend wird der pH Wert der Lösung mit 0,1 mol/l NaOH auf pH 8,30 eingestellt. Zu dieser Proteinlösung gibt man 370 mg Maleinimidohexanoyl-N-hydroxysuccinimid gelöst in 3 ml DMSO. Nach 15 minütiger Reaktionszeit bei Raumtemperatur wird der pH Wert mit 0,1 mol/l HCl auf pH 7,0 eingestellt und das Produkt über eine Gelpermeationschromatographie an Ultrogel AcA 202 (2 x 24 cm, Pharmacia, Schweden) aufgereinigt. Die Bestimmung der Anzahl an Maleinimidohexanoylgruppen im erhaltenen Produkt erfolgt gemäß EP-A 0 329 994 (Beispiel 4) mit Ellmann's Reagenz. In der Regel wird eine Beladung von 600 - 900 Maleinimidohexanoylgruppen je mol KLH erreicht.

Das so erhaltene KLH-Derivat wird ca. 15 min mit Argon gespült. Anschließend wird je mol Maleinimidohexanoylgruppe 2 mol des Peptid-Haptens zugegeben und 3 Stunden bei Raumtemperatur inkubiert. Die Aufreinigung erfolgt dann durch Abtrennung des nicht umgesetzten Peptids durch Gelpermeationschromatographie an Ultrogel® AcA 202 (2 x 24 cm, Pharmacia, Schweden).

**Beispiel 2:**

**Herstellung von polyklonalen Antiseren gegen HbA1$_c$**

**2.1 Immunisierung**

5 Schafe werden mit dem gemäß Beispiel 1.1 hergestellten Immunogen in Freund'schem Adjuvans immunisiert. Die Dosis beträgt jeweils 500 μg je Tier für die erste und jede weitere Folgeimmunisierung. 5 weitere Schafe wurden in derselben Weise immunisiert, jedoch mit jeweils 1 mg Immunogen je Tier und Immunisierung.

10 Schafe werden mit einem analogen Immunogen, bei dem das Hapten jedoch an KLH gebunden vorliegt (Herstellung gemäß Beispiel 1.2) in derselben Weise ebenfalls mit 500 μg bzw. 1 mg je Tier und Immunisierung immunisiert.

Nach 5 Monaten (KLH-Immunogen) bzw. 6 Monaten (β-Gal-Immunogen) werden allen Tieren Serumproben entnommen und die Serumstärke der erhaltenen Antiseren über einen Trübungstest bestimmt.

Antiserumprüfung

Verwendete Reagenzien:

Hämolysereagenz:

| 20 mM | MES pH 6,0 |
|---|---|
| 1 % | SDS |
| 0,02 % | Kaliumhexacyanoferrat (III) |
| 0,1 % | $NaN_3$. |
| 0,5 % | Brij® 35 |

Reaktionspuffer:

| 20 mM | MES pH 6,0 |
|---|---|
| 150 mM | NaCl |
| 0.5 % | Brij® 35 |
| 0,1 % | $NaN_3$ |
| 3 % | PEG 6000 |

Polyhaptenlösung:

| 20 mM | MES pH 6,0 |
|---|---|
| 150 mM | NaCl |
| 0,5 % | Brij® 35 |
| 0,1 % | $NaN_3$ |
| 6 % | PEG 6000 |
| 0,1 % | RSA |

Polyhapten 30 µg/ml

Vorbereitung der Antiseren zur Messung

Die Seren werden 1+1 mit doppelt konzentriertem Reaktionspuffer verdünnt, bei 4°C über Nacht inkubiert und der entstandene Niederschlag abzentrifugiert. Der Überstand wird 1 Stunde lang im Eisbad inkubiert, durch einen 0,22 µm-Filter filtriert und der pH-Wert auf 6,0 eingestellt. Die so entstandene Antikörperlösung kann direkt in den Test eingesetzt werden oder mit Reaktionspuffer weiter verdünnt werden.

Messung

Zur Beurteilung der Antiserumstärke wird Antikörperlösung und Polyhaptenlösung gemischt und die entstehende Trübung photometrisch gemessen. Die Messung wird bei 37°C am Analysenautomaten Hitachi 704 durchgeführt. Der Ablauf ist wie folgt: 8 µl Hämolysereagenz und 350 µl Antikörperlösung werden gemischt und 5 min lang inkubiert. Anschließend werden 70 µl Polyhaptenlösung hinzugefügt und weitere 5 min inkubiert. Die in dieser Zeit entstehende Trübung wird bei 340 nm (unter Verwendung der Referenzwellenlänge 700 nm) als Extinktion gemessen.

**2.3 Ergebnisse**

Die Ergebnisse sind in den Tabellen 1 (β-Gal-Immunogen) und 2 (KLH-Immunogen) zusammengefaßt. Bei niedrigen Antiserenverdünnungen (1:2 / 1:4) zeigen viele Tiere Meßsignale von 500 mE und mehr. Bei Antiserenverdünnung 1:8 trifft dies nur noch für ein Tier aus der β-Gal-Immunisierung zu, während es bei der Immunisierung mit KLH 8 Tiere sind. Die meisten dieser Seren erzeugen auch in dieser Verdünnung noch sehr hohe Meßsignale und lassen sich offensichtlich noch in hohen Verdünnungen zur Messung der glykierten Hämoglobine einsetzen. Die zur Immunisierung verwendete Dosis des Immunogens zeigt keinen erkennbaren Einfluß auf die Serumstärke.

Tabelle 1

| Maximales Meßsignal im kompetitiven Immunoassay bei Verwendung eines polyklonalen Antiserums, das durch Immunisierung mit einem an $\beta$-Galactosidase gebundenen Peptid-Hapten erhalten wurde | | | | |
|---|---|---|---|---|
| Tier | Dosis des Immunogens (mg) | Meßsignal mE bei Antiserumverdünnung 1:2 | Meßsignal mE bei Antiserumverdünnung 1:4 | Meßsignal mE bei Antiserumverdünnung 1:8 |
| 1 | 1.0 | 435 | 12 | 0 |
| 2 | 1,0 | 1261 | 309 | 0 |
| 3 | 1,0 | 77 | 2 | 0 |
| 4 | 1,0 | 205 | 2 | 0 |
| 5 | 1,0 | 887 | 305 | 4 |
| 6 | 0,5 | 1000 | 417 | 67 |
| 7 | 0,5 | 1289 | 992 | 650 |
| 8 | 0,5 | 1100 | 239 | 0 |
| 9 | 0,5 | 848 | 253 | 0 |
| 10 | 0,5 | 228 | 3 | 2 |

Tabelle 2

| Maximales Meßsignal im kompetitiven Immunoassay bei Verwendung eines polyklonalen Antiserums, das durch Immunisierung mit einem an KLH gebundenen Peptid-Hapten erhalten wurde | | | | |
|---|---|---|---|---|
| Tier | Dosis des Immunogens (mg) | Messignal mE bei Antiserumverdünnung 1:2 | Messignal mE bei Antiserumverdünnung 1:4 | Messignal mE bei Antiserumverdünnung 1:8 |
| 11 | 1,0 | 1580 | 1591 | 1575 |
| 12 | 1,0 | 1094 | 532 | 99 |
| 13 | 1,0 | 1044 | 844 | 356 |
| 14 | 1,0 | 1306 | 1548 | 1619 |
| 15 | 1,0 | 1620 | 1707 | 1696 |
| 16 | 0,5 | 1194 | 973 | 572 |
| 17 | 0,5 | 1435 | 1531 | 1394 |
| 18 | 0,5 | 1467 | 1598 | 1444 |
| 19 | 0,5 | 1158 | 1301 | 630 |
| 20 | 0,5 | 1486 | 1575 | 1608 |

**Beispiel 3:**

**Isolierung von $HbA_0$ und $HbS_0$ und in vitro Glykierung von $HbS_0$ zu $HbS1_c$**
Kommerziell erhältliches HbS (Sigma, Kat.-Nr. H0392) bzw. humanes Erythrozyten-Hämolysat wird chromatographisch getrennt und das so aufgereinigte $HbS_0$ in vitro mit Glucose zu $HbS1_c$ umgesetzt.

**3.1. Isolierung von $HbS_0$**

Ein HbS-Präparat der Fa. Sigma wurde in 50 mmol/1 MES pH 6,2 gelöst und anschließend gegen denselben Puffer 12 Stunden dialysiert. Das Dialysat wurde auf eine mit dem obigen Puffer equilibrierte S-Sepharose® HP Chromatographie-Säule der Fa. Pharmacia aufgetragen. Die Elution erfolgte durch einen LiCl-Gradienten. Die $HbS_0$-Fraktion wurde isoliert. Sie enthielt keine glykierten Hämoglobine, wie mittels HPLC-Analytik unter Verwendung einer $MonoS_{HbA1c}$-Säule der Fa. Pharmacia und deren Vorschrift zur Bestimmung von $HbA1_c$ gezeigt werden konnte.

**3.2. Herstellung von glykiertem HbS**

Ein Teil der HbS$_0$-Fraktion wurde gegen einen 100 mmol/l Phosphat-Puffer pH 6,5 dialysiert und anschließend mit einem 2600-fachen molaren Überschuß an Glucose umgesetzt. Nach 20 Stunden bei 37°C wurde die Reaktion abgebrochen und der Inkubationsansatz zur Entfernung der Glucose ausgiebig dialysiert. Das Dialysat wurde ebenfalls mittels HPLC analysiert. Das Chromatogramm wies einen Anteil von 11,1 % glykiertem HbS auf.

**3.3. Isolierung von HbA$_0$**

Durch Lyse von PBS gewaschenen Human-Erythrocyten in dest. Wasser wurden die enthaltenen Hämoglobine freigesetzt. Zelltrümmer und Erythrocyten-Ghosts wurden durch Zentrifugation abgetrennt. Das so erhaltene Hämolysat wurde gegen 50 mM MES pH 6,2 dialysiert. Das Dialysat wurde anschließend auf eine mit dem obigen Puffer equilibrierte S-Sepharose® HP Chromatographie-Säule der Fa. Pharmacia aufgetragen. Die Elution erfolgte durch einen LiCl-Gradienten. Die HbA$_0$-Fraktion wurde isoliert. Sie enthielt keine glykierten Hämoglobine, wie mittels HPLC-Analytik unter Verwendung einer MonoS/HbA1$_c$-Säule der Fa. Pharmacia und deren Vorschrift zur Bestimmung von HbA1$_c$ gezeigt werden konnte.

**Beispiel 4:**

**Simultane Bestimmung von HbA1$_c$ und HbS1$_c$**

Die Vollblut-Proben wurden zuerst mit einem speziellen Hämolysereagenz hämolysiert und anschließend an einem Photometer (BM/Hitachi 717 der Fa. Boehringer Mannheim GmbH) im Zweikanalverfahren vermessen. Im einen Kanal erfolgte der immunologische Nachweis von HbA1$_c$ in g/dl nach dem TINIA-Testprinzip durch turbidimetrische Messung bei 340 nm, während im anderen Kanal der Gesamthämoglobingehalt durch photometrische Messung bei 570 nm bestimmt wurde. Der prozentuale Gehalt an HbA1$_c$ wird nach folgender Formel berechnet:

$$\% \text{ HbA1}_c = \frac{\text{HbA1}_c \text{ [g/dl]}}{\text{Gesamthämoglobin [g/dl]}} \times 100$$

Folgende Reagenzien wurden verwendet:

1) Hämolysereagenz:

| | |
|---|---|
| 20 mM | Natriumphosphat pH 7,4 |
| 0,9 % | TTAB (Tetradecyltrimethylammoniumbromid) |

2) Antikörperlösung (R1 - HbA1$_c$):

| | |
|---|---|
| 20 mmol | MES-Puffer pH 6,2 (2-(N-Morpholino)-ethansulfonsäure) |
| 150 mmol | NaCl |
| 3,0 % | PEG 6000 (Polyethylenglycol MG ca. 6000) |
| 0,5 % | Brij® 35 |
| 1,0 mg/ml | PAK <HbA1$_c$>S-IgG(DE) (polyklonale Schafantikörper gegen HbA1$_c$), hergestellt mit Immunogen Fructose-Val-His-Leu-Thr-MH-βGal |

3) Polyhaptenlösung (R2 - HbA1$_c$):

| | |
|---|---|
| 20 mmol | MES-Puffer pH 6,2 |
| 150 mmol | NaCl |
| 6,0 % | PEG 6000 |
| 0,5 % | Brij® 35 |
| 30 μg/ml | Polyhapten[1] |

[1] Polyhapten: Fructose-Val-His-Leu-Thr, gekoppelt über Cys-Maleinimidohexansäure an Dextran wie in der deutschen Patentanmeldung P 41 40 142.5 beschrieben.

4) Pufferlösung zur Gesamthämoglobinbestimmung (R1 - Hb):

20 mM        Natriumphosphat pH 7,4
150 mMol    NaCl

5) Kalibratoren a - e:

20 mmol              Natriumphosphat pH 7,4
0,9 %                TTAB
1,4 mg/ml            Schafhämoglobin
0, 0,05, 0,1, 0,16, 0,3    humanes $HbA1_c$

### 4.1. Bestimmung von $HbA1_c$

Zur Probe wurde das Hämolysereagenz in einem Verhältnis von 1+100 hinzugefügt und bei 25°C ca. 5 Minuten inkubiert. Zu. 10 µl hämolysierter Probe wurden 250 µl Antikörperlösung (R1 - $HbA1_c$) hinzupipettiert. Nach 5 Minuten Inkubation bei 37°C wurde der Probenleerwert bei 700/340 nm bichromatisch gemessen (E1). Ca. 20 sec nach der Messung wurden 50 µl Polyhaptenlösung zugegeben, sofort gerührt und weitere 5 Minuten bei 37°C inkubiert. Danach wird die Trübung bei 700/340 nm bichromatisch gemessen (E2).

Die probenspezifische Extinktionsdifferenz wird nach der Formel

$$\Delta E = E_2 - K \cdot E_1$$

berechnet, wobei K den Volumenkorrekturfaktor darstellt.

$$K = \frac{V_{Probe} + V_{R1}}{V_{Gesamt}}$$

Mit Hilfe der Kalibratoren a - e, die aufsteigende Konzentrationen an $HbA1_c$ enthalten, wurde eine Eichkurve erstellt und über die probenspezifische Extinktionsdifferenz $\Delta E$ kann die unbekannte $HbA1_c$-Konzentration der Probe in g/dl abgelesen werden.

### 4.2. Bestimmung der Gesamthämoglobinkonzentration

Zur Bestimmung der Gesamthämoglobinkonzentration wird das in 4.1. erhaltene Hämolysat eingesetzt. Durch die im Hämolysereagenz enthaltenen Reagenzien wird das Hämoblogin oxidiert und durch Anlagerung des Detergenzmoleküls ein charakteristisches Hämoglobinchromophor enthalten. 20 µl dieses Hämolysats werden zusammen mit 230 µl Puffer (R1 - Hb) in die Küvette pipettiert, kurz gerührt und nach 5 Minuten Inkubation bei 37°C die Extinktion bichromatisch bei 660/570 nm gemessen. Mit Hilfe des Kalibrators a und phys. NaCl-Lösung (Nullstandard) wird eine Eichkurve erstellt und über die Extinktion der unbekannten Probe die Konzentration abgelesen.

Der prozentuale $HbA1_c$-Gehalt wird über die Formel

$$\% \, HbA1_c = \frac{HbA1_c \, [g/dl]}{Hb \, [g/dl]} \times 100$$

berechnet.

### Beispiel 5:

### Bestimmung der Spezifität der erfindungsgemäßen Antikörper

Zur Bestimmung der Spezifität der gemäß Beispiel 2 erhaltenen Antikörper wird die $HbA1_c$-Bestimmung gemäß Beispiel 4 am Hitachi 717 durchgeführt. Hierzu werden $HbA_0$ (nach Beispiel 3 aus Humanblut aufgereinigt), $HbS_0$ (Sigma H0392 gemäß Beispiel 3 aufgereinigt), $HbS1_c$-haltige Probe (in vitro gemäß Beispiel 3 hergestellt) und zwei Vollblutkontrollen mit bekannten $HbA1_c$-Werten (BioRad, Lyphochek® Diabetes Control Level 1 and 2, Best.-Nr. 740) als Proben vermessen. Das Ergebnis ist in der Tabelle 3 zusammengefaßt.

Tabelle 3

| Probe | HbA1$_c$/HbS1$_c$ [g/dl] | Hb [g/dl] | HbA1$_c$/HbS1$_c$ [%] |
|---|---|---|---|
| HbA$_0$ | 0 | 20 | 0 |
| HbS$_0$ | 0 | 13.9 | 0 |
| HbS$_0$ + HbS1$_c$ (HPLC: 11.1 %) | 1.33 | 12.3 | 10.8 |
| Lyphochek Level 1 (Sollwert 5.6 %) | 0.75 | 14.3 | 5.2 |
| Lyphochek Level 2 (Sollwert 10.4 %) | 0.98 | 9.1 | 10.8 |

Hieraus ergibt sich, daß die erfindungsgemäßen Antikörper sowohl HbA1$_c$ als auch HbS1$_c$ erkennen, nicht aber die entsprechenden nicht-glykierten Hämoglobine HbA$_0$ bzw. HbS$_0$.

**Beispiel 6:**

**Simultane Bestimmung von HbA1$_c$ und HbS1$_c$ in einer heterozygoten HbAS-Probe**

Die heterozygote HbAS-Probe (30 % HbS) wurde analog Beispiel 4 am Hitachi 717 vermessen. Vergleichend wurde der HbA1$_c$ -Gehalt mit einer hoch auflösenden HPLC-Methode nach Bisse E., Wieland H., J. Chromatogr. 434, 1988, 95 - 110 (Elutionsprofil siehe Figur 1) und der Glykohämoglobin-Gehalt mit der affinitätschromatographischen Bestimmungsmethode Glyc-Affin der Firma IsoLab (Best.-Nr. SG 6200) bestimmt. Die Ergebnisse sind in Tabelle 4 zusammengefaßt:

Tabelle 4

| | HbA1$_c$ | HbS1$_c$ | HbA1c + HbS1$_c$ |
|---|---|---|---|
| Immunoassay | - | - | 5,6 % |
| HPLC | 3,3 % | - | - |
| Glyc-Affin | - | - | 5,4 % |

Mit der HPLC-Methode nach Bisse und Wieland wird ein HbA1$_c$-Wert von 3,3 % gemessen. Der Wert für glykiertes HbS (HbS1$_c$) kann nicht angegeben werden, da die Elutionszeit des HbS1$_c$ -Peaks nicht exakt bekannt ist. Es könnte sich um einen der Peaks bei 31 - 33 Minuten Elutionszeit handeln.

Durch die Erfassung von HbS1$_c$ mißt die Tinaquant-Test um 2,3 % höher als die HPLC-Methode. Mit der affinitätschromatographischen Methode (Erfassung von Glykohämoglobin = HbA1$_c$ + HbS1$_c$ + HbA1$_a$ + HbA1$_b$ + ε-Lysin-glyk. Hb) werden 7,25 % Glykohämoglobin gemessen. Aufgrund der Miterfassung dieser weiteren glykierten Hämoglobinspezies mißt der Glyc-Affin-Test höher als der beschriebene immunologische Test. Im Methodenvergleich mit normalen Proben zwischen Tinaquant und Glyc-Affin ergibt sich deshalb eine Ausgleichsgerade von

% HbA1$_c$ (Immunoassay) = 0.66 x
% Glykohämoglobin(Affinitätschromatographie) + 0,6

mit einer sehr guten Korrelation (Figur 2). Wird nun der Glyc-Affin-Wert mit Hilfe der Formel der Ausgleichsgeraden um die Erfassung von HbA1$_a$, HbA1$_b$ etc. korrigiert, dann ergibt sich ein nahezu identischer Meßwert für HbA1$_c$ + HbS1$_c$ mit dem Immunoassay.

**Patentansprüche**

1.    Verwendung von Antikörpern, die HbA1$_c$, HbS1$_c$ und HbC1$_c$ erkennen und erhältlich sind durch Immunisierung mit mindestens einem Immunogen, welches als Haptenteil das glykierte Oligopeptid Fructose-Val-His, Fructose-Val-His-Leu, Fructose-Val-His-Leu-Thr und/oder Fructose-Val-His-Leu-Thr-Pro enthält, zur immunologischen, simultanen Bestimmung von HbA1$_c$, HbS1$_c$, und HbC1$_c$.

2.    Verwendung gemäß Anspruch 1, **dadurch gekennzeichnet, dass** die immunologische Bestimmung nach dem Prinzip des Agglutinationstests oder nach den Cloned Enzyme Donor Immunoassay, dem Enzyme- Multiplied-Immunoassay, dem Fluoreszenz-Polarisations-Immunoassay oder dem ELISA-(enzyme linked immuno sorbent assay)-Prinzip erfolgt.

**3.** Verwendung von Antikörpern gemäß einem der Ansprüche 1 oder 2, **dadurch gekennzeichnet, dass** die Antikörper erhältlich sind durch Immunisierung eines Säugers mit einem Gemisch aus einem Immunogen, das als Hapten Fructose-Val-His-Leu-Thr enthält und mindestens einem weiteren Immunogen, welches als Haptenteil Fructose-Val-His, Fructose-Val-His-Leu und/oder Fructose-Val-His-Leu-Thr-Pro enthält, und Isolierung des Antikörpers aus dem Serum der immunisierten Tiere.

**4.** Verwendung von Antikörpern gemäß einem der Ansprüche 1 oder 2, **dadurch gekennzeichnet, dass** der Antikörper ein monoklonaler Antikörper ist und erhältlich ist durch Immunisierung mit mindestens einem Immunogen, das als Haptenteil das glykierte Oligopeptid Fructose-Val-His, Frutose-Val-His-Leu und/oder Fructose-Val-His-Thr-Pro enthält, Immortalisierung der Milzzellen der immunisierten Tiere, Klonierung derjenigen immortalisierten Zellen, die den gewünschten Antikörper produzieren und Isolierung des Antikörpers nach bekannten Verfahren.

**Claims**

**1.** Use of antibodies which recognize $HbA1_c$, $HbS1_c$ and $HbC1_c$ and are obtainable by immunization with at least one immunogen which contains the glycated oligopeptide fructose-Val-His, fructose-Val-His-Leu, fructose-Val-His-Leu-Thr and/or fructose-Val-His-Leu-Thr-Pro as a hapten component, for the immunological simultaneous determination of $HbA1_c$, $HbS1_c$ and $HbC1_c$.

**2.** Use as claimed in claim 1, wherein the immunological determination is carried out according to an agglutination test principle or according to the cloned enzyme donor immunoassay, enzyme multiplied immunoassay, fluorescence polarisation immunoassay or ELISA (enzyme-linked immunosorbent assay) technique.

**3.** Use of antibodies as claimed one of the claims 1 or 2, wherein the antibodies are obtainable by immunizing a mammal with a mixture of an immunogen that contains fructose-Val-His-Leu-Thr as the hapten and with at least one further immunogen that contains fructose-Val-His, fructose-Val-His-Leu and/or fructose-Val-His-Leu-Thr-Pro as the hapten component and isolating the antibody from the serum of the immunized animals.

**4.** Use of antibodies as claimed in one of the claims 1 or 2, wherein the antibody is a monoclonal antibody and is obtainable by immunizing with at least one immunogen that contains the glycated oligopeptide fructose-Val-His, fructose-Val-His-Leu and/or fructose-Val-His-Leu-Thr-Pro as the hapten component, immortalizing the spleen cells of the immunized animals, cloning those immortalized cells which produce the desired antibody and isolating the antibody according to known methods.

**Revendications**

**1.** Utilisation d'anticorps qui reconnaissent $HbA1_c$, $HbS1_c$ et $HbC1_c$ et qui peuvent être obtenus par immunisation avec au moins un immunogène qui contient, à titre de fraction haptène, l'oligopeptide glycosylé fructose-Val-His, fructose-Val-His-Leu, fructose-Val-His-Leu-Thr et/ou fructose-Val-His-Leu-Thr-Pro, pour la détermination immunologique simultanée de $HbA1_c$, de $HbS1_c$ et de $HbC1_c$.

**2.** Utilisation selon la revendication 1, **caractérisée en ce que** la détermination immunologique est effectuée suivant le principe du test d'agglutination ou suivant le dosage immunologique de type cloned enzyme donor immunoassay, le dosage immunologique de type enzyme multiplied immunoassay, le dosage immunologique par polarisation de fluorescence ou suivant le principe ELISA (enzyme linked immuno sorbent assay).

**3.** Utilisation d'anticorps selon l'une des revendications 1 ou 2, **caractérisée en ce que** les anticorps peuvent être obtenus par immunisation d'un mammifère avec un mélange d'un immunogène qui contient, à titre d'haptène, le fructose-Val-His-Leu-Thr et d'au moins un immunogène supplémentaire qui contient, à titre de fraction haptène, le fructose-Val-His, le fructose-Val-His-Leu et/ou le fructose-Val-His-Leu-Thr-Pro et par isolement de l'anticorps du sérum des animaux immunisés.

**4.** Utilisation d'anticorps selon l'une des revendications 1 ou 2, **caractérisée en ce que** l'anticorps est un anticorps monoclonal et **en ce qu'**il peut être obtenu par immunisation avec au moins un immunogène qui contient, à titre de fraction haptène, l'oligopeptide glycosylé fructose-Val-His, fructose-Val-His-Leu et/ou fructose-Val-His-Leu-Thr-Pro, par immortalisation des cellules de rate des animaux immunisés, par clonage des cellules immortalisées qui produisent l'anticorps souhaité et par isolement de l'anticorps suivant des procédés connus.